# EUROPEAN PATENT APPLICATION

(11) **EP 4 728 971 A1**
(43) Date of publication of application: **22.04.2026**
(21) Application number: 25208969.3
(22) Date of filing: 15.10.2025
(51) Int. Cl.: A61B 5/00, A61B 5/145, A61B 5/1486, A61B 5/1495, G16H 40/40, A61B 5/1473

(54) **METHOD FOR CALIBRATING AND VERIFYING IMPLANTABLE DETECTING DEVICE AND IMPLANTABLE DETECTING DEVICE**

(30) Priority: 16.10.2024 CN 202411446018
(71) Applicant: Shenzhen Goodix Technology Co., Ltd., Shenzhen, Guangdong 518045 (CN)
(72) Inventor: LIU, Haifeng, Shenzhen, 518045 (CN); CHENG, Shuqing, Shenzhen, 518045 (CN)
(74) Representative: Kraus & Lederer PartGmbB

(57) **Abstract**

A method for calibrating and/or verifying an implantable detecting device and an implantable detecting device are provided. The method includes: outputting a square wave signal to an implantable detecting device that has been implanted into a subject, where the implantable detecting device is configured to detect a concentration of an analyte in the subject, and a potential of the square wave signal increases incrementally over time; receiving a first current signal produced by the implantable detecting device in response to the square wave signal; generating a calibration curve based on a correspondence between the first current signal and the square wave signal; and performing calibration and/or functional verification on the implantable detecting device based on the calibration curve.

## Description

### TECHNICAL FIELD

Embodiments of the present application relate to the technical field of data processing, and in particular, to a method for calibrating and/or verifying an implantable detecting device and an implantable detecting device.

### BACKGROUND

With improvement of people's living standards, an incidence rate of diabetes is also becoming higher, and people pay more attention to blood glucose testing. Blood glucose testing, as an important way of comprehensive management of diabetes, plays an extremely important role in diabetes diagnosis, diabetes control, and diabetes treatment. Long-term continuous dynamic blood glucose testing can effectively reduce or delay occurrence of diabetes complications, improve the quality of life of patients. A continuous glucose monitoring system can continuously monitor blood glucose for 7 days, 10 days, or 14 days. However, in a long-term monitoring process, electrode passivation, enzyme activity attenuation, and enzyme and electronic medium loss, functional film layer expansion, or the like may occur to a biological enzyme and a functional film layer in a sensor in the continuous glucose monitoring system and cause a change in sensitivity of the sensor to glucose detection, thereby leading to inaccurate detection results of the continuous glucose monitoring system.

Currently, in a process of producing blood glucose testing sensors, a manufacturer performs a large quantity of tests on sensors of a same production process to acquire an experience curve of sensor sensitivity changing with time, and then inserts the experience curve into a continuous glucose monitoring system. When the continuous glucose monitoring system is used, the experience curve is invoked to automatically adjust the sensitivity of a sensor.

However, performance differences exist between different sensors, and consistency differences between the sensors lead to relatively large errors of blood glucose testing results after the sensors are calibrated by using the experience curve.

### SUMMARY

In view of this, embodiments of the present application provide a method for calibrating and/or verifying an implantable detecting device, an implantable detecting device, an apparatus, and an electronic device, to at least partially resolve the foregoing problems.

According to a first aspect of the embodiments of the present application, a method for calibrating and/or verifying an implantable detecting device is provided, and the method is applied to an apparatus for calibrating and/or verifying an implantable detecting device, and includes: outputting a square wave signal to an implantable detecting device that has been implanted into a subject, where the implantable detecting device is configured to detect a concentration of an analyte in the subject, and a potential of the square wave signal increases incrementally over time; receiving a first current signal produced by the implantable detecting device in response to the square wave signal; generating a calibration curve based on a correspondence between the first current signal and the square wave signal; and performing calibration and/or functional verification on the implantable detecting device based on the calibration curve.

According to a second aspect of the embodiments of the present application, an implantable detecting device is provided, including: a first substrate, a first functional film layer, and a first electrode group formed on the first substrate, the first electrode group including a first working electrode, a first counter electrode, and a first reference electrode, and the first functional film layer covering the first substrate and the first electrode group.

According to a third aspect of the embodiments of the present application, an implantable detecting device is provided, including: a second substrate, a second functional film layer, and a second electrode group formed on the second substrate, the second electrode group including a second working electrode, a third working electrode, a second counter electrode, and a second reference electrode, and the second functional film layer covering the second substrate and the second electrode group.

According to a fourth aspect of the embodiments of the present application, an apparatus for calibrating and/or verifying an implantable detecting device is provided, including: an output unit, configured to output a square wave signal to an implantable detecting device that has been implanted into a subject, where the implantable detecting device is configured to detect a concentration of an analyte in the subject, and a potential of the square wave signal increases incrementally over time; a receiving unit, configured to receive a first current signal produced by the implantable detecting device in response to the square wave signal; a generation unit, configured to generate a calibration curve based on a correspondence between the first current signal and the square wave signal; and a calibrating and verifying unit, configured to perform calibration and/or functional verification on the implantable detecting device based on the calibration curve.

According to a fifth aspect of the embodiments of the present application, an electronic device is provided, including: the apparatus for calibrating and/or verifying an implantable detecting device described in the fourth aspect.

According to the method for calibrating and/or verifying an implantable detecting device provided in an embodiment of the present application, a square wave signal is output to an implantable detecting device that has been implanted into a subject, a first current signal produced by the implantable detecting device in response to the square wave signal is received, and a calibration curve is generated based on a correspondence between the first current signal and the square wave signal. Therefore, calibration and/or functional verification may be performed on the implantable detecting device based on the calibration curve, calibration may be performed on the implantable detecting device that has been implanted into the subject, and different calibrations may be performed on different implantable detecting devices. Performance differences of different implantable detecting devices are considered during calibration. Compared with performing calibration on an implantable detecting device based on an experience curve in the conventional technology, the implantable detecting device after calibration can more accurately detect a concentration of an analyte, and functional verification may be performed on the implantable detecting device based on the calibration curve. Therefore, an operating state of the implantable detecting device may be determined, and a relatively large concentration detection error of the analyte caused by a defect of the implantable detecting device may be prevented, thereby improving working stability of the implantable detecting device is improved.

### BRIEF DESCRIPTION OF DRAWINGS

To describe the technical solutions in the embodiments of the present application or in the conventional technology more clearly, the following briefly describes the accompanying drawings for describing the embodiments or the conventional technology. It is clear that the accompanying drawings in the following description are merely some embodiments recorded in the embodiments of the present application, and those of ordinary skill in the art may still derive other drawings from these accompanying drawings.
FIG. 1 is a flowchart of a method for calibrating and/or verifying an implantable detecting device according to an embodiment of the present application;
FIG. 2 is a schematic diagram of a square wave signal according to an embodiment of the present application;
FIG. 3 is a flowchart of a calibration method according to an embodiment of the present application;
FIG. 4 is a schematic diagram of a reference curve according to an embodiment of the present application;
FIG. 5 is a schematic diagram of sensitivity attenuation according to an embodiment of the present application;
FIG. 6 is a schematic diagram of a calibration curve in a hydration process according to an embodiment of the present application;
FIG. 7 is a schematic diagram of a correspondence between a square wave signal and a calibration curve according to an embodiment of the present application;
FIG. 8 is a schematic diagram of an implantable detecting device according to an embodiment of the present application;
FIG. 9 is a schematic diagram of another implantable detecting device according to an embodiment of the present application; and
FIG. 10 is a schematic diagram of an apparatus for calibrating and/or verifying an implantable detecting device according to an embodiment of the present application.

### DETAILED DESCRIPTION

To enable those skilled in the art to better understand the technical solutions in the embodiments of the present application, the following clearly and completely describes the technical solutions in the embodiments of the present application with reference to the accompanying drawings in the embodiments of the present application. It is clear that the described embodiments are only a part rather than all of the embodiments of the present application. Based on the embodiments in the embodiments of the present application, all other embodiments obtained by those of ordinary skill in the art shall fall within the protection scope of the embodiments of the present application.

The terms used in the present application are for the sole purpose of describing specific embodiments and are not intended to limit the present application. The terms "one", "the", and "this" of singular forms used in the present application and the appended claims are also intended to include plural forms, unless otherwise specified in the context clearly. It should be further understood that the term "and/or" used herein indicates and includes any or all possible combinations of one or more associated listed items.

It should be understood that although the terms "first", "second", "third", and the like may be used in the present application to describe various types of information, the information should not be limited to these terms. These terms are only used to distinguish between information of a same type. For example, without departing from the scope of the present application, "first information" may also be referred to as "second information", and similarly, "second information" may also be referred to as "first information". Depending on the context, for example, the word "if" used herein can be interpreted as "while", "when", or "in response to determining".

As mentioned above, with improvement of people's living standards, an incidence rate of diabetes is also becoming higher, and people pay more attention to blood glucose testing. Blood glucose testing, as an important way of comprehensive management of diabetes, plays an extremely important role in diabetes diagnosis, diabetes control, and diabetes treatment. Long-term continuous dynamic blood glucose testing can effectively reduce or delay occurrence of diabetes complications, improve the quality of life of patients. A continuous glucose monitoring system can continuously monitor blood glucose for 7 days, 10 days, or 14 days. However, in a long-term monitoring process, electrode passivation, enzyme activity attenuation, and enzyme and electronic medium loss, functional film layer expansion, or the like may occur to a biological enzyme and a functional film layer in a sensor in the continuous glucose monitoring system and cause a change in sensitivity of the sensor to glucose detection, thereby leading to inaccurate detection results of the continuous glucose monitoring system. Currently, in a process of producing blood glucose testing sensors, a manufacturer performs a large quantity of tests on sensors of a same production process to acquire an experience curve of sensor sensitivity changing with time, and then inserts the experience curve into a continuous glucose monitoring system. When the continuous glucose monitoring system is used, the experience curve is invoked to automatically adjust the sensitivity of a sensor. However, performance differences exist between different sensors, and consistency differences between the sensors lead to relatively large errors of blood glucose testing results after the sensors are calibrated by using the experience curve.

An embodiment of the present application provides a method for calibrating and/or verifying an implantable detecting device. In the method, a square wave signal is output to an implantable detecting device that has been implanted into a subject, a first current signal produced by the implantable detecting device in response to the square wave signal is received, and a calibration curve is generated based on a correspondence between the first current signal and the square wave signal. Therefore, calibration and/or functional verification may be performed on the implantable detecting device based on the calibration curve, calibration may be performed on the implantable detecting device that has been implanted into the subject, and different calibrations may be performed on different implantable detecting devices. Performance differences of different implantable detecting devices are considered during calibration. Compared with performing calibration on an implantable detecting device based on an experience curve in the conventional technology, the implantable detecting device after calibration can more accurately detect a concentration of an analyte, and functional verification may be performed on the implantable detecting device based on the calibration curve. Therefore, an operating state of the implantable detecting device may be determined, and a relatively large concentration detection error of the analyte caused by a defect of the implantable detecting device may be prevented, thereby improving working stability of the implantable detecting device is improved.

The following describes, by using embodiments, a method for calibrating and/or verifying an implantable detecting device provided in the present application.

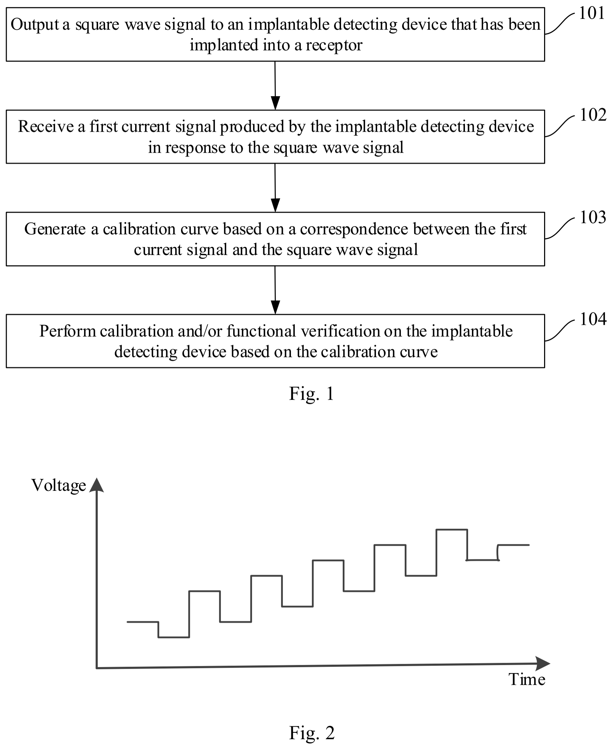

FIG. 1 is a flowchart of a method for calibrating and/or verifying an implantable detecting device according to an embodiment of the present application. The method for calibrating and/or verifying the implantable detecting device is applied to an apparatus for calibrating and/or verifying an implantable detecting device. As shown in FIG. 1, the method for calibrating and/or verifying the implantable detecting device includes the following step 101 to step 104:
Step 101. Output a square wave signal to an implantable detecting device that has been implanted into a subject.

The implantable detecting device may detect a concentration of an analyte in the subject. The analyte may be a substance in a subject body such as blood glucose, blood ketone, uric acid, or lactic acid. The square wave signal is output to the implantable detecting device that has been implanted into the subject. A potential of the square wave signal increases incrementally over time, that is, a low-level voltage and a high-level voltage of the square wave signal gradually increase in each cycle. In an example, FIG. 2 is a schematic diagram of a square wave signal according to an embodiment of the present application. In FIG. 2, the horizontal coordinate represents a time, the vertical coordinate represents a voltage value. As shown in FIG. 2, a low level and a high level of the square wave signal increases incrementally over time .

Step 102. Receive a first current signal produced by the implantable detecting device in response to the square wave signal.

A square wave voltage is applied to the implantable detection device, and under excitation of the square wave voltage, characteristic substances or groups on the implantable detecting device undergo rapid an electrochemical reaction on a working electrode to produce the first current signal.

Step 103. Generate a calibration curve based on a correspondence between the first current signal and the square wave signal.

Because the potential of the square wave signal increases incrementally over time and the potential of the square wave signal is different, the first current signal produced by the implantable detecting device is different. Therefore, the calibration curve is produced based on a correspondence between the potential of the square wave signal and the first current signal, and the calibration curve may indicate a relationship between a voltage and a current.

Step 104. Perform calibration and/or functional verification on the implantable detecting device based on the calibration curve.

The implantable detecting device is calibrated based on the calibration curve. For example, the detecting sensitivity of the implantable detecting device may be adjusted based on the calibration curve, and/or functional verification may be performed on the implantable detecting device based on the calibration curve. For example, whether the implantable detecting device is damaged, operates normally, completes initialization, or the like is determined based on the calibration curve.

In this embodiment of this application, a square wave signal is output to an implantable detecting device that has been implanted into a subject, a first current signal produced by the implantable detecting device in response to the square wave signal is received, and a calibration curve is generated based on a correspondence between the first current signal and the square wave signal. Therefore, calibration and/or functional verification may be performed on the implantable detecting device based on the calibration curve, calibration may be performed on the implantable detecting device that has been implanted into the subject, and different calibrations may be performed on different implantable detecting devices. Performance differences of different implantable detecting devices are considered during calibration. Compared with performing calibration on an implantable detecting device based on an experience curve in the conventional technology, the implantable detecting device after calibration can more accurately detect a concentration of an analyte, and functional verification may be performed on the implantable detecting device based on the calibration curve. Therefore, an operating state of the implantable detecting device may be determined, and a relatively large concentration detection error of the analyte caused by a defect of the implantable detecting device may be prevented, thereby improving operating stability of the implantable detecting device is improved.

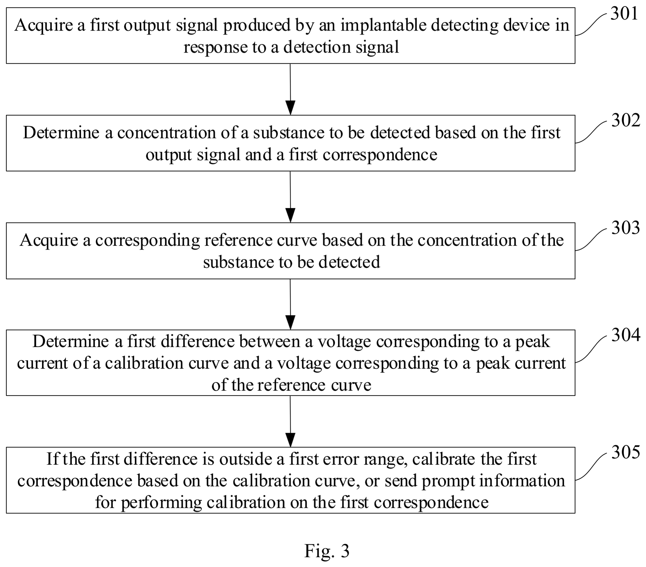

FIG. 3 is a flowchart of a calibration method according to an embodiment of the present application. As shown in FIG. 3, when calibration is performed on an implantable detecting device based on a calibration curve, the following step 301 to step 305 may be performed:

Step 301. Acquire a first output signal produced by the implantable detecting device in response to a detection signal.

The detection signal is output to the implantable detecting device, and the detection signal may be a constant-voltage signal. After receiving the detection signal, the implantable detecting device detects a concentration of an analyte in a subject based on the detection signal, to produce the first output signal.

Step 302. Determine the concentration of the analyte based on the first output signal and a first correspondence.

The first correspondence may indicate a relationship between the output signal produced by the implantable detecting device in response to the detection signal and the concentration of the analyte. It should be understood that the concentration of the analyte is in direct proportion to the first output signal, that is, a higher concentration of the analyte indicates a higher signal strength of the first output signal. The first correspondence may be a correspondence preset by a manufacturer of the implantable detecting device.

Step 303. Acquire a corresponding reference curve based on the concentration of the analyte.

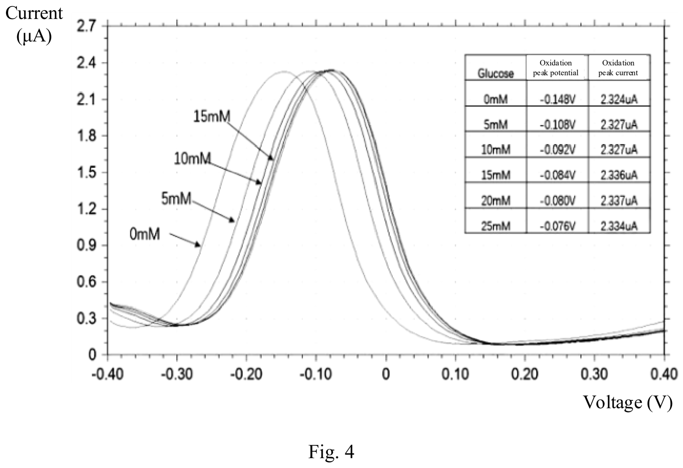

A reference curve corresponding to the concentration of the analyte is determined based on the detected concentration of the analyte. It should be understood that, when the voltage is consistent, a higher concentration of the analyte indicates a higher signal strength of the first output signal. FIG. 4 is a schematic diagram of the reference curve provided in this embodiment of the present application. In FIG. 4, the horizontal coordinate represents a voltage and the vertical coordinate represents a current. As shown in FIG. 4, that a concentration of glucose in a blood is detected by the implantable detecting device is used as an example. When glucose exists in the blood, an enzyme in the implantable detecting device catalyzes the glucose to generate electrons which are transferred to an electron mediator in the implantable detecting device, the electron mediator losses electrons on an electrode surface in the implantable detecting device, so that it is easy to implement an oxidation-reduction reaction. Therefore, when the concentration of glucose on the electrode surface of the implantable detecting device is higher, a bio-enzyme layer of the implantable detecting device oxidizes the glucose to generate more electrons, and then the electron mediator on the electrode surface can obtain electrons more easily and then be oxidized. Therefore, a voltage corresponding to a peak current of an oxidation reaction of the implantable detecting device moves to 0 V with the increase of the concentration of glucose. As shown in FIG. 4, the concentration of glucose has a significant effect on a voltage corresponding to a peak current of the oxidation-reduction reaction in the reference curve. With the increasing concentration of glucose, the voltage corresponding to the peak current of the oxidation-reduction reaction in the reference curve gradually shifts to a positive potential. However, because a current magnitude of the peak current of the oxidation-reduction reaction in the reference curve of the implantable detecting device is related to a concentration of the electron mediator on the electrode surface, when the electron mediator on the electrode surface is in a stable or saturated state, there is no significant effect on the current magnitude of the peak current of the oxidation-reduction reaction in the reference curve. Therefore, the current magnitude of the peak current of the oxidation-reduction reaction is not affected by the concentration of glucose. It should be understood that the above is only an example for convenience of explanation, and should not impose any limitation on this embodiment of the present application.

Step 304. Determine a first difference between a voltage corresponding to a peak current of the calibration curve and a voltage corresponding to a peak current of the reference curve.

The glucose in the foregoing example is used for description. A square wave signal is output to the implantable detecting device that has been implanted into the subject, a first current signal generated by the implantable detecting device in response to the square wave signal is received, a calibration curve is generated based on a correspondence between the first current signal and the square wave signal, and a voltage corresponding to a peak current in the calibration curve is determined. In this case, assuming that the concentration of glucose is 10 mM, subtraction is performed between a voltage corresponding to a peak current of a reference curve corresponding to the concentration of glucose of 10 mM and the voltage corresponding to the peak current in the calibration curve, to determine the first difference between the voltage corresponding to the peak current of the calibration curve and the voltage corresponding to the peak current of the reference curve.

Step 305: If the first difference is outside a first error range, calibrate the first correspondence based on the calibration curve, or send a notification for performing calibration on the first correspondence.

The first error range may be set as required. When the first difference is outside the first error range, it is proved that a deviation of detecting the concentration of the analyte by the implantable detecting device is relatively large. In this case, calibration is performed on the first correspondence based on the calibration curve, that is, calibration is performed on the correspondence between the output signal of the implantable detecting device and the concentration of the analyte. In an example, calibration may be performed on the first correspondence based on the calibration curve by using a pretrained calibration model.

Alternatively, a notification may be sent. The a notification may indicate that the implantable detecting device needs to be calibrated, and an operator may be prompted by the notification to calibrate the first correspondence.

In this embodiment of the present application, a first output signal produced by the implantable detecting device in response to a detection signal is acquired, the concentration of the analyte is determined based on the first output signal and the first correspondence, and a corresponding reference curve is acquired based on the concentration of the analyte. If a first difference between the voltage corresponding to the peak current of the reference curve and the voltage corresponding to the peak current of the calibration curve is outside the first error range, calibration is performed on the first correspondence based on the calibration curve. Therefore, automatic calibration may be performed on the implantable detecting device when it is detected that a deviation between the concentration of the analyte and a real concentration is relatively large. Because whether the implantable detecting device needs calibration is automatically detected based on a difference between the voltage corresponding to the peak current of the reference curve and the voltage of corresponding to the peak current of the calibration curve, automatic calibration may be performed on the implantable detecting device or the operator may be notified to manually calibrating the implantable detecting device when a detection deviation of the implantable detecting device is relatively large, so that the implantable detecting device can more accurately detect the concentration of the analyte.

In a possible implementation, when calibration is performed on the implantable detecting device based on the calibration curve, a historical calibration curve previously generated after a square wave signal is output to the implantable detecting device may be acquired, and a second difference between the peak current of the calibration curve and a peak current of the historical calibration curve is determined. If the second difference is outside a second error range, calibration is performed on the implantable detecting device based on the calibration curve.

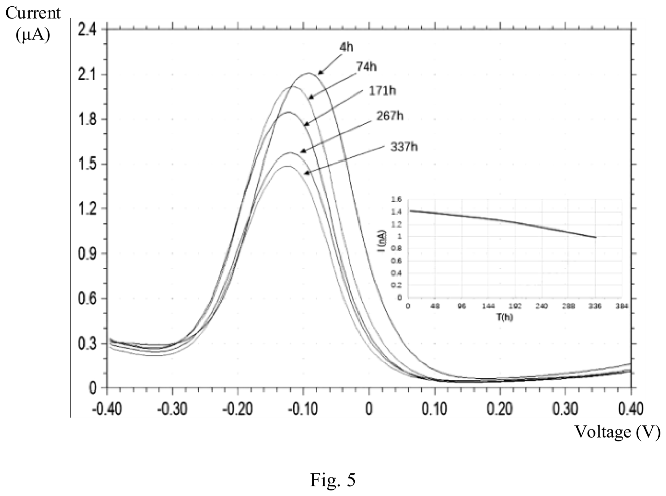

First, that the analyte is glucose is used as an example to describe a sensitivity change of the implantable detection device. In a short time in a use process of the implantable detection device, for example, in 12h, 6h, or 3h, the sensitivity of the implantable detection device to glucose is almost unchanged, and an oxidation-reduction peak current magnitude of the calibration curve remains unchanged, and is not affected to change by a concentration change of glucose. However, after the implantable detecting device works for a long time, for example, works for 24h, 48h, or 72h, the enzyme layer activity decreases due to loss of an enzyme layer on the implantable detecting device, and an electron transfer capability decreases due to slow loss of characteristic substances or groups on an electrode, which will cause a decrease in detection sensitivity of the implantable detecting device to glucose. That is, a signal strength of a current signal output by the implantable detecting device at a same concentration of glucose decreases. FIG. 5 is a schematic diagram of sensitivity attenuation according to an embodiment of the present application. In FIG. 5, the horizontal coordinate represents voltage, the vertical coordinate represents current, T(h) represents time, and I(nA) represents current. As shown in FIG. 5, the implantable detecting device is tested in a glucose buffer of 10mM for 337h, that is, undergoes a sensitivity test for 14 days. A square wave signal is output to the implantable detecting device at different time points such as after 4h, 74h, 171h, 267h, and 337h, to generate a plurality of calibration curves. As shown in FIG. 5, it may be found that magnitudes of oxidation-reduction peak currents of the calibration curves corresponding to different time points decrease with time, from about 2.1 uA to about 1.5 uA, and attenuate by 28.57%, which indicates that the enzyme layer and characteristic substances or groups having electrochemical activity on the implantable detecting device are lost to different degrees. In addition, because of the loss of the enzyme layer and the characteristic substances or groups having electrochemical activity, the sensitivity of the implantable detecting device to glucose decreases from about 0.14 nA/mM to 0.1 nA/mM, and the sensitivity decreases by about 28.6%. It can be learned that a magnitude change of the oxidation-reduction peak currents of the calibration curves of the implantable detecting device has a clear correspondence with a change of the sensitivity of the implantable detecting device working for a long time to glucose.

When calibration is performed on the implantable detecting device based on the calibration curve, a historical calibration curve previously generated based on the square wave signal output and the received first current signal after the square wave signal is output to the implantable detecting device may be acquired, a second difference between a magnitude of a peak current of a calibration curve generated after a square wave signal is output to the implantable detecting device this time and a magnitude of the peak current of the historical calibration curve is determined. If the second difference is outside the second error range, it is determined that the sensitivity of the implantable detecting device is greatly attenuated. In this case, calibration is performed on the implantable detecting device based on the calibration curve. Optionally, when calibration is performed on the implantable detecting device based on the calibration curve, automatic calibration may be performed based on the calibration curve by using a calibration model, or a notification indicating a need for performing manual calibration on the first correspondence relationship may be sent to the operator.

Optionally, a square wave signal may be output to the implantable detecting device periodically, to detect a sensitivity attenuation degree of the implantable detecting device periodically, or a condition for outputting a square wave signal to the implantable detecting device may be set, and when the condition is met, a square wave signal is output to the implantable detecting device. The condition may be set as required, for example, the concentration of the analyte is greater than a concentration threshold. Glucose is used as an example for description. When a calibration curve of the implantable detecting device is acquired, that is, when a square wave signal is output to the implantable detecting device, a relatively smooth state of a blood glucose change needs to be selected, for example, 3 hours before breakfast or after a meal, to avoid an interference to the first current signal due to a large blood glucose fluctuation caused by eating or strenuous exercise. Therefore, a change range of the blood glucose may be set as a condition for outputting a square wave signal to the implantable detecting device.

In this embodiment of the present application, the historical calibration curve generated after a square wave signal is output to the implantable detecting device last time is acquired, and the second difference between the peak current of the calibration curve and the peak current of the historical calibration curve is determined. If the second difference is outside the second error range, calibration is performed on the implantable detecting device based on the calibration curve. Therefore when the sensitivity of the implantable detecting device attenuates greatly, calibration may be performed on the implantable detecting device in a timely manner, so that relatively low accuracy of detecting, by the implantable detecting device, the concentration of the analyte due to sensitivity attenuation of the implantable detecting device may be prevented, and the implantable detecting device can more accurately detect the concentration of the analyte.

In a possible implementation, when a calibration curve is generated for the first time after the implantable detecting device is implanted into the subject, if a waveform of the calibration curve conforms to a predetermined waveform pattern when functional verification is performed on the implantable detecting device based on the calibration curve, a peak current of the calibration curve is within a reference current range, and a voltage corresponding to the peak current is within a reference voltage range, it is determined that a function of the implantable detecting device is normal.

When a calibration curve is generated for the first time after the implantable detecting device is implanted into the subject for the first time, it may be determined whether a waveform of the calibration curve conforms to a predetermined waveform pattern, for example, whether the waveform of the calibration curve conforms to the waveform pattern shown in FIG. 4 and FIG. 5, that is, whether to meet: when a voltage of a square wave signal changes from negative to positive, a current changes from high to low and then from low to high, and then decreases symmetrically to reach a smooth state after reaching a peak value. In addition, whether a magnitude of the peak current of the calibration curve is within a reference current range, and whether a voltage corresponding to the peak current is within a reference voltage range are determined, for example, whether the magnitude of the peak current is located in (2.4 µA, 2.7 µA), and whether the voltage corresponding to the peak current is located in (-0.3 V, -0.2 V) are determined to determine whether a function of the implantable detection device is normal.

It should be noted that a current generated by the implantable detecting device at an initial stage after the implantable detecting device is implanted into the subject is affected by a film layer hydration state, and changes greatly. In addition, whether an electrode of the implantable detecting device is damaged in an implantation process, whether a function of a reference electrode is normal, or the like cannot be determined by using a current magnitude. In addition, a correct calibration curve can be determined only when the reference electrode, a working electrode, and a counter electrode of the implantable detecting device are normal. Therefore, whether a function of the implantable detecting device after implantation is normal may be determined based on whether a shape of the calibration curve conforms to a predetermined waveform pattern, whether a peak current of the calibration curve is within a reference current range, and whether a voltage corresponding to the peak current is within a reference voltage range.

In this embodiment of the present application, when a calibration curve is generated for the first time after the implantable detecting device is implanted into the subject for the first time, if a waveform of the calibration curve complies with the waveform change rule and a peak current of the calibration curve is within a peak current range, it is determined that the function of the implantable detecting device is normal. After the implantable detecting device is implanted into the subject for the first time, functional verification may be performed on the implantable detecting device, to determine whether the implantable detecting device is damaged, and prevent low accuracy of detecting a concentration of an analyte due to damage of the implantable detecting device, thereby avoiding an adverse effect on a wearer.

In a possible implementation, when functional verification is performed on the implantable detecting device based on a calibration curve, the kth calibration curve generated after a square wave signal is output to the implantable detecting device for the kth time may be acquired, where k is an integer greater than 1. A first duration between a time point at which the implantable detecting device is implanted into the subject and a time point at which the kth calibration curve is acquired is determined. If the first duration is less than a duration threshold, a voltage corresponding to a peak current in the kth calibration curve is within a voltage range, and voltages corresponding to peak currents in the first to the (k-1)th calibration curves are outside the voltage range, it is determined that initialization of the implantable detecting device is completed, so that a detection signal can be output to the implantable detecting device after the initialization of the implantable detecting device is completed.

Within a preset time threshold, a square wave signal is output to the implantable detecting device for a plurality of times, and the implantable detecting device generates a plurality of calibration curves based on the plurality of square wave signals.

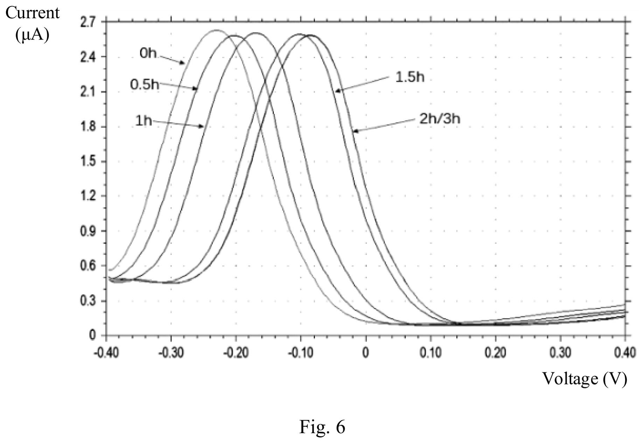

The following describes an initialization process of the implantable detecting device by using glucose as an analyte. After the implantable detecting device is implanted into the subject, a hydration process is performed. The hydration process is specifically as follows: A functional film layer of a working area of the implantable detecting device contacts interstitial fluid, water molecules and glucose in the interstitial fluid penetrate into the functional film layer of the implantable detecting device, and undergo an electrochemical reaction. In this case, the film layer transforms from a dry film to a wet film, activity of an enzyme layer and a glucose transmission capability of the functional layer are unstable. The hydration process generally takes 1 hour to 2 hours, or longer. In this case, a square wave signal is output to the implantable detecting device, and a calibration curve is generated. A change of the calibration curve in the hydration process may reflect a hydration state of the implantable detecting device. FIG. 6 is a schematic diagram of a calibration curve in a hydration process according to an embodiment of the present application. In FIG. 6, the horizontal coordinate represents voltage, and the vertical coordinate represents current. As shown in FIG. 6, as the implantable detecting device is implanted into the subject, the hydration process starts. Voltages corresponding to oxidation-reduction peak currents of calibration curves corresponding to the implantable detecting device when hydration lasts 0h, 0.5h, 1h, 1.5h, 2h, and 3h gradually move toward a positive potential, the movement of the voltages corresponding to the oxidation-reduction peak currents of the calibration curves is related to an electron transfer speed on an electrode surface, and the electron transfer speed on the electrode surface is closely related to an ion environment and a mass transfer speed on the electrode surface. The hydration process of the implantable detecting device is a process of rebuilding an ion environment on a surface of a working electrode of the implantable detecting device, and the extension and expansion of the functional film layer in contact with water leads to a change in the mass transfer speed. Therefore, changes of the voltages corresponding to the oxidation-reduction peak currents in the calibration curves on the implantable detecting device can reflect a hydration degree of the implantable detecting device. As shown in FIG. 6, calibration curves of 2h and 3h of the implantable detecting device overlap, which indicates that the hydration process of the implantable detecting device is completed after hydration is performed for 2h, the ion environment and the mass transfer speed on the electrode surface of the implantable detecting device remains stable, and electrochemical performance of the implantable detecting device is in a stable state.

It may be learned from the foregoing example that, when functional verification is performed on the implantable detecting device based on the calibration curve, the kth calibration curve generated after a square wave signal is output to the implantable detecting device for the kth time may be acquired. If the voltage corresponding to the peak current of the kth calibration curve is within the voltage range and the voltages corresponding to the peak currents of the first to the (k-1)th calibration curves are outside the voltage range, it is determined that the initialization of the implantable detecting device is completed, that is, hydration is completed. For example, in FIG. 6, a calibration curve corresponding to 2h is the kth calibration curve, calibration curves corresponding to 0h, 0.5h, 1h, and 1.5h are the first to the (k-1)th calibration curves, the voltage corresponding to the peak current of the kth calibration curve is within a voltage range (-110 mV, 0 mV), and the voltages corresponding to the peak currents of the first to the (k-1)th calibration curves are outside the voltage range (-110 mV, 0 mV), which proves that the initialization of the implantable detecting device is completed, that is, hydration is completed. In this case, a detection signal may be output to the implantable detecting device, to perform calibration on the implantable detecting device or detect a concentration of an analyte by using the implantable detecting device. It should be noted that the duration threshold is generally greater than a time point at which the hydration of the implantable detecting device is completed. When the first duration is within the duration threshold, if the voltage corresponding to the peak current of the calibration curve meets a voltage range requirement, the initialization of the implantable detecting device is completed.

In this embodiment of the present application, the kth calibration curve generated after a square wave signal is output to the implantable detecting device for the kth time is acquired. If the first duration between the time point at which the kth calibration curve is acquired and a time point at which the implantable detecting device is implanted is less than the duration threshold, the voltage corresponding to the peak current in the kth calibration curve is within the voltage range, and the voltages corresponding to the peak currents in the first to the (k-1)th calibration curves are outside the voltage range, it is determined that the initialization of the implantable detecting device is completed. Therefore, the initialization process of the implantable detecting device may be checked, and calibration may be performed on the implantable detecting device in a timely manner after the initialization process of the implantable detecting device is completed, so that when the chemical state of the implantable detecting device is unstable, the implantable detecting device is prevented from being calibrated, to prevent an error in a relationship between a current signal output by the implantable detecting device after calibration and the concentration of the analyte, which enables the implantable detecting device to more accurately detect the concentration of the analyte.

In a possible implementation, when functional verification is performed on the implantable detecting device based on the calibration curve, if a voltage corresponding to a peak current in a final calibration curve is outside the voltage range, after calibration is performed on the first correspondence based on the final calibration curve, it is determined that the initialization of the implantable detecting device is completed, to output the detection signal to the implantable detecting device after the initialization of the implantable detecting device is completed, where the final calibration curve is the last calibration curve acquired in the first duration.

If the voltage corresponding to the peak current of the last calibration curve acquired in the first duration is outside the voltage range, and voltages corresponding to peak currents of all calibration curves acquired in the first duration are outside the voltage range, it is determined that the hydration process of the implantable detecting device is completed. However, sensitivity of the implantable detecting device is relatively low due to a defect of the implantable detecting device or another reason, that is, the voltage corresponding to the peak current of the calibration curve of the implantable detecting device cannot reach the voltage range. In this case, calibration may be performed on the implantable detecting device by using the final calibration curve, that is, the last obtained calibration curve, so that the implantable detecting device can detect the concentration of the analyte. The initialization of the implantable detecting device after calibration is determined as completed, so that the implantable detecting device can work normally. It should be noted that the calibration method in any one of the foregoing embodiments may be performed by performing calibration on the implantable detecting device based on the final calibration curve, and details are not described herein again.

In this embodiment of the present application, if the voltage corresponding to the peak current of the last calibration curve acquired in the first duration is outside the voltage range, after calibration is performed on the first correspondence based on the last calibration curve, it is determined that the initialization of the implantable detecting device is completed. Therefore, the implantable detecting device whose sensitivity is reduced due to the defect of the implantable detecting device or another reason can normally detect the concentration of the analyte, and utilization of the implantable detecting device can be improved.

In a possible implementation, the implantable detecting device includes a first substrate, a first functional film layer, and a first electrode group formed on the first substrate. The first electrode group includes a first working electrode, a first counter electrode, and a first reference electrode. The first functional film layer covers the first substrate and the first electrode group. When a square wave signal is output to the implantable detecting device that has been implanted into the subject, the output of the detection signal to the first working electrode may be stopped, and a square wave signal is output to the first working electrode.

Because the first working electrode of the implantable detecting device including three electrodes receives the detection signal, to produce a first output signal to detect the concentration of the analyte, when a square wave signal is sent to the implantable detecting device including the three electrodes, the output of the detection signal to the first working electrode needs to be stopped and then a square wave signal is sent to the first working electrode after the output of the detection signal is stopped. Optionally, because the detection signal (with a constant voltage potential) on the first working electrode is switched to the square wave signal, an algorithm may be used to eliminate interference caused by voltage switching of the first working electrode of the implantable detecting device to stability of the detection signal with a constant voltage potential.

In this embodiment of the present application, for the implantable detecting device including the three electrodes, when a square wave signal is output to the implantable detecting device, the output of the detection signal to the first working electrode may be stopped, and a square wave signal is output to the first working electrode. Therefore, a square wave signal can be output to the implantable detecting device, and interference of a calibration curve generating process to the detection signal with a constant voltage potential may be prevented.

In a possible implementation, the implantable detecting device includes a second substrate, a second functional film layer, and a second electrode group formed on the second substrate. The second electrode group includes a second working electrode, a third working electrode, a second counter electrode, and a second reference electrode. The second functional film layer covers the second substrate and the second electrode group. When a square wave signal is output to the implantable detecting device that has been implanted into the subject, the square wave signal may be output to one of the second working electrode and the third working electrode, where the other of the second working electrode and the third working electrode receives a detection signal.

For an implantable detecting device including four electrodes, that is, an implantable detecting device including a second working electrode and a third working electrode, a square wave signal may be output to one of the second working electrode and the third working electrode, and a detection signal may be output to the other of the second working electrode and the third working electrode. Therefore, the concentration of the analyte may be detected by using one working electrode, and generation of a calibration curve may be performed by using the other working electrode. Optionally, a detection signal may be output to both the second working electrode and the third working electrode, and when a calibration curve needs to be generated, a square wave signal may be output to one of the second working electrode and the third working electrode after stopping outputting the detection signal. Therefore, when a calibration curve does not need to be generated, the concentration of the analyte is detected by using two working electrodes, so that detection accuracy may be improved.

In this embodiment of the present application, for the implantable detecting device including four electrodes, when a square wave signal is output to the implantable detecting device, the square wave signal may be output to one of the second working electrode and the third working electrode, and a detection signal may be output to the other of the second working electrode and the third working electrode. Therefore, the square wave signal is output to the implantable detecting device, and the concentration of the analyte can be detected while a calibration curve is generated. In addition, a reference curve needs to be determined by using the concentration of the analyte when calibration is performed on the implantable detecting device, and by simultaneously generating a calibration curve and detecting the concentration of the analyte, it may be determined that the concentration of the analyte may be detected while the calibration curve is generated. Compared with detecting the concentration first and then generating the calibration curve in the solution of three electrodes in the foregoing embodiment, the calibration process may be more accurate. In addition, a detection signal with a constant voltage potential and a square wave calibration signal are acquired on different working electrodes, so that signal interference between them can be avoided.

In a possible implementation, a frequency range of the square wave signal is [10 Hz-10 KHz], a voltage increment per cycle of the square wave signal is in the range of (0, 10 mV], and a signal amplitude range of the square wave signal is (0, 50 mV].

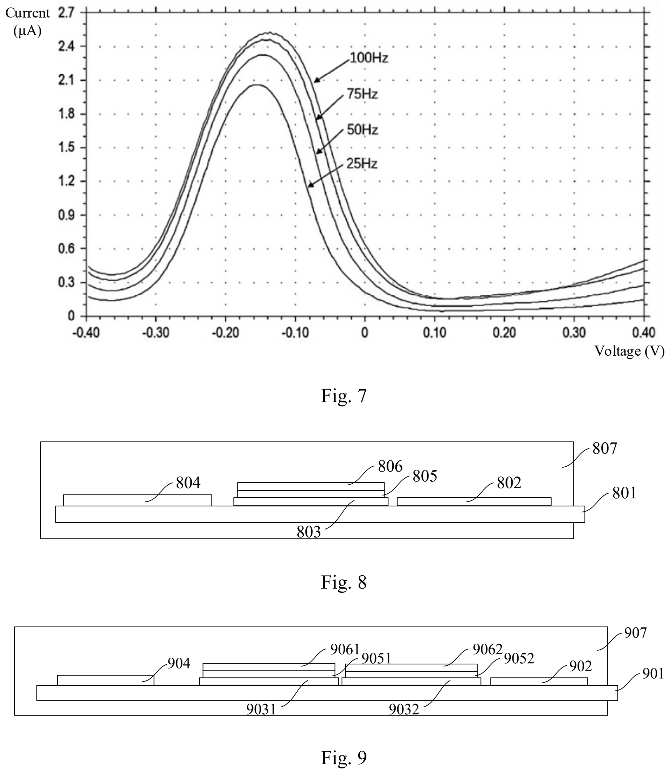

FIG. 7 is a schematic diagram of a correspondence between a square wave signal and a calibration curve according to an embodiment of the present application. In FIG. 7, the horizontal coordinate represents voltage, and the vertical coordinate represents current. As shown in FIG. 7, peak currents of calibration curves formed at different frequencies of a square wave signal are different. A higher frequency of the square wave signal leads to a higher generated peak current. In addition, a current unit of the peak currents shown in FIG. 7 is a micro-ampere level, and is greater than a first output signal (about 0.5-100 nano-amperes) output by a current implantable detecting device. Therefore, the calibration curves have excellent identifiability.

In this embodiment of the present application, a frequency range of the square wave signal is [10 Hz-10 KHz], a cycle increment range of the square wave signal is (0, 10 mV], and a signal amplitude range of the square wave signal is (0, 50 mV]. Therefore, the square wave signal may be set as required, and a square wave signal whose potential increases incrementally over time e may be applied to the implantable detecting device.

FIG. 8 is a schematic diagram of an implantable detecting device according to an embodiment of the present application. As shown in FIG. 8, the implantable detecting device includes: a first substrate 801, a first functional film layer 807, and a first electrode group formed on the first substrate 801. The first electrode group includes a first working electrode 803, a first counter electrode 804, and a first reference electrode 802. The first functional film layer 807 covers the first substrate 801 and the first electrode group.

The first substrate 801 may be a flexible base material. A plurality of electrodes are prepared on a single side of the first substrate 801 by using a screen printing technology, a spray printing technology, or the like, to form an independent electrochemical three-electrode system, where the first working electrode 803, the first counter electrode 804, and the first reference electrode 802 constitute a first electrode group formed on the first substrate 801. In an example, as shown in FIG. 8, a characteristic substance or group is located in a film layer 805 above the first working electrode 803. In another example, the characteristic substance or group may be located inside the first working electrode 803, for example, a material of the first working electrode 803. Optionally, an enzyme layer 806 may be located above the film layer 805, or an enzyme and the characteristic substance or group may be mixed into one layer. The characteristic substance or group mainly serves as an electron transfer material of the enzyme layer 806, to transfer electrons generated by a reaction of the enzyme layer 806 with the analyte to the first working electrode 803. The first functional film layer 807 having transmission and biocompatibility of the analyte covers the entire three-electrode system.

In this embodiment of the present application, the implantable detecting device includes: the first substrate 801, the first electrode group formed on the first substrate 801, and the first functional film layer 807. The first electrode group includes the first working electrode 803, the first counter electrode 804, and the first reference electrode 802. The first functional film layer 807 covers the first substrate 801 and the first electrode group. Therefore, the concentration of the analyte may be detected by the implantable detecting device.

FIG. 9 is a schematic diagram of another implantable detecting device according to an embodiment of the present application. As shown in FIG. 9, the implantable detecting device includes a second substrate 901, a second functional film layer 907, and a second electrode group formed on the second substrate 901. The second electrode group includes a second working electrode 9031, a third working electrode 9032, a second counter electrode 904, and a second reference electrode 902. The second functional film layer 907 covers the second substrate 901 and the second electrode group.

The second substrate 901 may be a flexible base material. A plurality of electrodes are prepared on a single side of the second substrate 901 by using a screen printing technology, a spray printing technology, or the like, to form an independent electrochemical four-electrode system, where the second working electrode 9031, the third working electrode 9032, the second counter electrode 904, and the second reference electrode 902 may constitute a first electrode group formed on the first substrate. In an example, as shown in FIG. 9, a characteristic substance or group is located in a film layer 9051 above the second working electrode 9031 and a film layer 9052 above the third working electrode 9032. In another example, the characteristic substance or group may be located inside the second working electrode 9031 and the third working electrode 9032, for example, working electrode materials. Optionally, an enzyme layer 9061 may be located in the film layer 9051, and an enzyme layer 9062 may be located above the film layer 9052, or an enzyme and the characteristic substance or group may be mixed into one layer. The characteristic substance or group mainly serves as an electron transfer material of the enzyme layer 9061 and the enzyme layer 9062, to transfer electrons generated by a reaction of the enzyme layer and the analyte to a working electrode. The second functional film layer 907 having transmission and biocompatibility of the analyte covers the entire four-electrode system.

In this embodiment of the present application, the implantable detecting device includes the second substrate 901, the second functional film layer 907, and the second electrode group formed on the second substrate 901. The second electrode group includes the second working electrode 9031, the third working electrode 9032, the second counter electrode 904, and the second reference electrode 902. The second functional film layer 907 covers the second substrate 901 and the second electrode group. Therefore, the concentration of the analyte can be simultaneously detected by using two working electrodes, and detection precision of the concentration of the analyte can be improved.

It should be noted that for a method for calibrating and/or verifying the implantable detecting device having three electrodes and the implantable detecting device having four electrodes in the foregoing embodiments, reference may be made to the method performed in any one of the foregoing embodiments, and details are not described herein again.

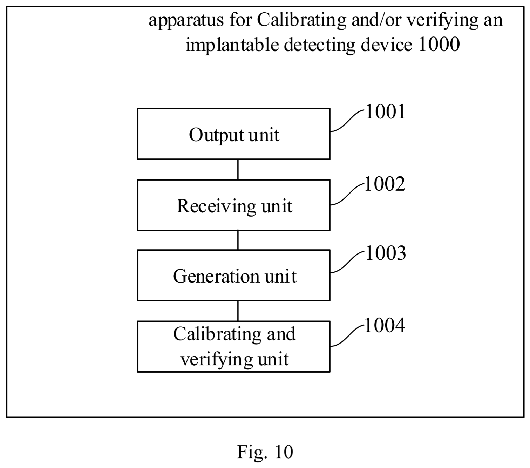

FIG. 10 is a schematic diagram of an apparatus for calibrating and/or verifying an implantable detecting device according to an embodiment of the present application. As shown in FIG. 10, the apparatus 1000 includes: an output unit 1001, configured to output a square wave signal to an implantable detecting device that has been implanted into a subject, where the implantable detecting device is configured to detect a concentration of a substance to be tested in the subject, and a potential of the square wave signal increases incrementally over time; a receiving unit 1002, configured to receive a first current signal produced by the implantable detecting device in response to the square wave signal; a generation unit 1003, configured to generate a calibration curve based on a correspondence between the first current signal and the square wave signal; and a calibration and verification unit 1004, configured to perform calibration and/or functional verification on the implantable detecting device based on the calibration curve.

In this embodiment of the present application, the output unit 1001 may be configured to perform Step 101 in the foregoing method embodiment, the receiving unit 1002 may be configured to perform Step 102 in the foregoing method embodiment, the generation unit 1003 may be configured to perform Step 103 in the foregoing method embodiment, and the calibration and verification unit 1004 may be configured to perform Step 105 in the foregoing method embodiment.

In a possible implementation, the calibration and verification unit 1004 may be further configured to: acquire a first output signal that is produced by the implantable detecting device in response to a detection signal; determine a concentration of an analyte based on the first output signal and a first correspondence, where the first correspondence is used to indicate a relationship between the output signal produced by the implantable detecting device in response to the detection signal and the concentration of the analyte; acquire a corresponding reference curve based on the concentration of the analyte; determine a first difference between a voltage corresponding to a peak current of the calibration curve and a voltage corresponding to a peak current of the reference curve; and if the first difference is outside a first error range, calibrate the first correspondence based on the calibration curve, or send a notification for performing calibration on the first correspondence.

In a possible implementation, the calibration and verification unit 1004 may be further configured to: acquire a historical calibration curve generated after a square wave signal is output to the implantable detecting device last time; determine a second difference between the peak current of the calibration curve and a peak current of the historical calibration curve; and if the second difference is outside a second error range, perform calibration on the implantable detecting device based on the calibration curve.

In a possible implementation, the calibration and verification unit 1004 may be further configured to: when the calibration curve is generated for the first time after the implantable detecting device is implanted into the subject, if a waveform of the calibration curve complies with a waveform change rule, a peak current is within a reference current range, and a voltage corresponding to the peak current is within a reference voltage range, determine that a function of the implantable detecting device is normal.

In a possible implementation, the calibration and verification unit 1004 may be further configured to: acquire the kth calibration curve generated after a square wave signal is output to the implantable detecting device for the kth time, where k is an integer greater than 1; determine a first duration between a time point at which the implantable detecting device is implanted into the subject and a time point at which the kth calibration curve is acquired; and if the first duration is less than a duration threshold, a voltage corresponding to a peak current in the kth calibration curve is within a voltage range, and voltages corresponding to peak currents in the first to the (k-1)th calibration curves are outside the voltage range, determine that initialization of the implantable detecting device is completed, so that a detection signal can be output to the implantable detecting device after the initialization of the implantable detecting device is completed.

In a possible implementation, the calibration and verification unit 1004 may be further configured to: if a voltage corresponding to a peak current in a final calibration curve is outside the voltage range, after calibration is performed on the first correspondence based on the final calibration curve, determine that the initialization of the implantable detecting device is completed, to output the detection signal to the implantable detecting device after the initialization of the implantable detecting device is completed, where the final calibration curve is the last calibration curve acquired in the first duration.

In a possible implementation, the implantable detecting device includes: a first substrate, a first functional film layer, and a first electrode group formed on the first substrate. The first electrode group includes a first working electrode, a first counter electrode, and a first reference electrode. The first functional film layer covers the first substrate and the first electrode group. The output unit 1001 may be further configured to: stop outputting the detection signal to the first working electrode, and output a square wave signal to the first working electrode.

In a possible implementation, the implantable detecting device includes: a second substrate, a second functional film layer, and a second electrode group formed on the second substrate. The second electrode group includes a second working electrode, a third working electrode, a second counter electrode, and a second reference electrode. The second functional film layer covers the second substrate and the second electrode group. The output unit 1001 may be further configured to: output a square wave signal to one of the second working electrode and the third working electrode, where the other of the second working electrode and the third working electrode receives a detection signal.

In a possible implementation, a frequency range of the square wave signal is [10 Hz-10 KHz], a voltage increment per cycle of the square wave signal is in the range of (0, 10 mV], and a signal amplitude range of the square wave signal is (0, 50 mV].

An embodiment of the present application further provides an electronic device. The electronic device includes an apparatus 1000 for calibrating and/or verifying an implantable detecting device in the foregoing embodiment. The apparatus 1000 for calibrating and/or verifying an implantable detecting device may perform the method for calibrating and/or verifying an implantable detecting device in any one of the foregoing embodiments.

It should be noted that, according to an implementation requirement, the components/steps described in the embodiments of the present application may be split into more components/steps, or two or more components/steps or the components/some operations of the steps may be combined into new components/steps, to implement the objectives of the embodiments of the present application.

The foregoing method according to the embodiments of the present application may be implemented in hardware or firmware, or is implemented as software or computer code that may be stored in a recording medium (such as a CD ROM, a RAM, a floppy disk, a hard disk, or a magnetooptical disk), or is implemented as computer code that is downloaded through a network and originally stored in a remote recording medium or a non-temporary machine-readable medium and that is to be stored in a local recording medium. Therefore, the method described herein may be processed by such software stored on a recording medium that uses a general-purpose computer, a dedicated processor, or a programmable or dedicated hardware (such as an ASIC or a FPGA). It may be understood that a computer, a processor, a microprocessor controller, or programmable hardware includes a storage component that can store or receive software or computer code (for example, a RAM, a ROM, or a flash memory). When the software or computer code is accessed and executed by the computer, the processor, or hardware, the method for calibrating and/or verifying an implantable detecting device described herein is implemented. In addition, when a general-purpose computer accesses the code for implementing the method for calibrating and/or verifying an implantable detecting device shown herein, execution of the code converts the general-purpose computer into a special-purpose computer for performing the method for calibrating and/or verifying an implantable detecting device shown herein.

A person of ordinary skill in the art may be aware that the units and the method steps in the examples described with reference to the embodiments disclosed in this specification can be implemented by using electronic hardware or a combination of computer software and electronic hardware. Whether these functions are implemented by hardware or software depends on particular applications and design constraints of the technical solutions. A person skilled in the art may implement the described functions for each particular application by using different methods, but such implementation should not be considered beyond the scope of the embodiments of the present application.

The foregoing implementations are merely used to describe the embodiments of the present application, but are not intended to limit the embodiments of the present application. A person of ordinary skill in the art may make various changes and variations without departing from the spirit and scope of the embodiments of the present application. Therefore, all equivalent technical solutions also fall within the scope of the embodiments of the present application. The patent protection scope of the embodiments of the present application shall be defined by the claims.

## Claims

1. A method for calibrating and/or verifying an implantable detecting device, applied to an apparatus for calibrating and/or verifying an implantable detecting device, the method comprising:
outputting a square wave signal to an implantable detecting device that has been implanted into a subject, wherein the implantable detecting device is configured to detect a concentration of an analyte in the subject, and a potential of the square wave signal increases incrementally over time;
receiving a first current signal produced by the implantable detecting device in response to the square wave signal;
generating a calibration curve based on a correspondence between the first current signal and the square wave signal; and
performing calibration and/or functional verification on the implantable detecting device based on the calibration curve.

2. The method according to claim 1, wherein the performing calibration on the implantable detecting device based on the calibration curve comprises:
acquiring a first output signal produced by the implantable detecting device in response to a detection signal;
determining the concentration of the analyte based on the first output signal and a first correspondence, wherein the first correspondence is used to indicate a relationship between an output signal produced by the implantable detecting device in response to a detection signal and the concentration of the analyte;
acquiring a corresponding reference curve based on the concentration of the analyte;
determining a first difference between a voltage corresponding to a peak current of the calibration curve and a voltage corresponding to a peak current of the reference curve; and
if the first difference is outside a first error range, calibrating the first correspondence based on the calibration curve, or sending notification for calibrating the first correspondence.

3. The method according to claim 1, wherein the performing calibration on the implantable detecting device based on the calibration curve comprises:
acquiring a historical calibration curve previously generated after the square wave signal is output to the implantable detecting device;
determining a second difference between a peak current of the calibration curve and a peak current of the historical calibration curve; and
if the second difference is outside a second error range, calibrating the implantable detecting device based on the calibration curve.

4. The method according to claim 1, wherein when the calibration curve is generated for the first time after the implantable detecting device is implanted into the subject, the performing functional verification on the implantable detecting device based on the calibration curve comprises:
if a waveform of the calibration curve conforms to a predetermined waveform pattern, a peak current of the calibration curve is within a reference current range, and a voltage corresponding to the peak current is within a reference voltage range, determining that a function of the implantable detection device is normal.

5. The method according to claim 2, wherein the performing functional verification on the implantable detecting device based on the calibration curve comprises:
acquiring a kth calibration curve generated after a square wave signal is output to the implantable detecting device for a kth time, wherein k is an integer greater than 1;
determining a first duration between a time at which the implantable detecting device is implanted into the subject and a time at which the kth calibration curve is acquired; and
if the first duration is less than a duration threshold, a voltage corresponding to a peak current in the kth calibration curve is within a voltage range, and voltages corresponding to a 1st calibration curve to a (k-1)th calibration curve are outside the voltage range, determining that initialization of the implantable detecting device is completed, to output the detection signal to the implantable detecting device after the initialization of the implantable detecting device is completed.

6. The method according to claim 5, wherein the performing functional verification on the implantable detecting device based on the calibration curve comprises:
if a voltage corresponding to a peak current in a final calibration curve is outside the voltage range, after calibrating the first correspondence based on the final calibration curve, determining that the initialization of the implantable detecting device is completed, to output the detection signal to the implantable detecting device after the initialization of the implantable detecting device is completed, wherein the final calibration curve is the last calibration curve acquired in the first duration.

7. The method according to claim 2, wherein the implantable detecting device comprises: a first substrate, a first functional film layer, and a first electrode group formed on the first substrate, the first electrode group comprising a first working electrode, a first counter electrode, and a first reference electrode, and the first functional film layer covering the first substrate and the first electrode group; and
the outputting a square wave signal to an implantable detecting device that has been implanted into a subject comprises:
stopping outputting the detection signal to the first working electrode, and outputting the square wave signal to the first working electrode.

8. The method according to claim 2, wherein the implantable detecting device comprises: a second substrate, a second functional film layer, and a second electrode group formed on the second substrate, the second electrode group comprising a second working electrode, a third working electrode, a second counter electrode, and a second reference electrode, and the second functional film layer covering the second substrate and the second electrode group; and
the outputting a square wave signal to an implantable detecting device that has been implanted into a subject comprises:
outputting the square wave signal to one of the second working electrode and the third working electrode, wherein the detection signal is received by the other of the second working electrode and the third working electrode.

9. The method according to any one of claims 1 to 8, wherein a frequency range of the square wave signal is [10 Hz-10 KHz], a voltage increment per cycle of the square wave signal is in a range of (0, 10 mV], and a signal amplitude range of the square wave signal is (0, 50 mV].

10. An implantable detecting device, comprising: a first substrate, a first functional film layer, and a first electrode group formed on the first substrate, the first electrode group comprising a first working electrode, a first counter electrode, and a first reference electrode, and the first functional film layer covering the first substrate and the first electrode group.
